(19) Europäisches Patentamt
European Patent Office
Office européen des brevets

(11) Veröffentlichungsnummer: **0 004 929**
**B1**

(12) **EUROPÄISCHE PATENTSCHRIFT**

(45) Veröffentlichungstag der Patentschrift: **08.04.81**

(21) Anmeldenummer: **79101085.3**

(22) Anmeldetag: **09.04.79**

(51) Int. Cl.³: **A 01 N 43/30, A 01 N 43/90, C 07 D 493/04**

(54) **Isochroman-derivate enthaltende insektizide und akarizide Mittel, Verfahren zu ihrer Herstellung und ihre Verwendung.**

(30) Priorität: **15.04.78 DE 2816475**

(43) Veröffentlichungstag der Anmeldung:
**31.10.79 Patentblatt 79/22**

(45) Bekanntmachung des Hinweises auf die Patenterteilung:
**08.04.81 Patentblatt 81/14**

(84) Benannte Vertragsstaaten:
**BE CH DE FR GB IT NL SE**

(56) Entgegenhaltungen:
**US - A - 4 066 648**

**JOURNAL OF ORGANIC CHEMISTRY, Vol. 27, Nr. 10, Oktober 10. 1962, J.N. SRIVASTAVA et al.: "Dihydroisocoumarins", Seiten 4337—4341**

(73) Patentinhaber: **BAYER AG**
**Zentralbereich Patente, Marken und Lizenzen**
**D-5090 Leverkusen 1, Bayerwerk (DE)**

(72) Erfinder: **Mues, Volker, Dr.**
**Marèestrasse 61**
**D-5600 Wuppertal 1 (DE)**
Erfinder: **Behrenz, Wolfgang, Dr.**
**Untergründemich 14**
**D-5063 Overath (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 004 929 B1

Isochroman-derivate enthaltende insektizide und akarizide Mittel, Verfahren zu ihrer Herstellung und ihre Verwendung

Die Erfindung betrifft neue Isochroman-derivate enthaltende insektizide und akarizide Mittel, Verfahren zur ihrer Herstellung sowie ihre Verwendung zur Bekämpfung von Insekten und Spinnentieren.

Es ist bereits bekannt, daß die folgenden Wirkstoffe bzw. Wirkstoffgruppen pestizide, insbesondere insektizide und akarizide Eigenschaften besitzen:

A) Carbamate, wie z.B. das 2-iso-Propoxy-phenyl-N-methyl-carbamat, 3,4,5-Trimethyl-phenyl-N-methyl-carbamat, 1-Naphthyl-N-methyl-carbamat, 2,3-Dihydro-2,2-dimethyl-7-benzofuranyl-N-methyl-carbamat, 2-(1,3-Dioxolan(2)yl-phenyl)-N-methyl-carbamat und 2,2-Dimethyl-1,3-benzodioxol(4)yl-N-methyl-carbamat,

B) Carbonsäureester, wie z.B. 2,3,4,5-Tetrahydrophthalimido-methyl-chrysanthemat und (5-Benzyl-3-furyl)-methyl-2,2-dimethyl-3-(2-methyl-propenyl)-cyclopropancarboxylat,

C) Phosphorsäureester, wie z.B. O,O-Dimethyl-O-(2,2-dichlorvinyl)-phosphorsäureester und

D) Halogenalkane, wie z.B. 1,1,1-Trichlor-2,2-bis-(4-methoxyphenyl)-äthan und 1,1,1-Trichlor-2,2-bis-(4-chlorphenyl)-äthan.

Weiterhin sind synergistische Mischungen von Carbamaten, z.B. 2-iso-Propoxy-phenyl-N-methyl-carbamat oder von Phosphorsäureestern, z.B. O,O-Diäthyl-O-(2-iso-propyl-4-methylpyrimidin(6)yl)-thionophosphorsäureester oder von natürlichen oder synthetischen Pyrethroiden mit Piperonyläthern, wie z.B. $\alpha$-(2-(2-Butoxy-äthoxy)-äthoxy)-4,5-methylendioxy-2-propyl-toluol, bekannt (vergleiche Bull. Org. mond. Santé/Bull. Wld. Hlth Org.1966, *35* 691—708; Schrader, G.: Die Entwicklung neuer insektizider Phosphorsäureester 1963, S.158). Doch ist die Wirksamkeit dieser synergistischen Wirkstoffkombinationen nicht befriedigend. Eine gewisse praktische Bedeutung hat bisher nur das $\alpha$-(2-(2-Butoxy-äthoxy)-äthoxy)-4,5-methylendioxy-2-propyl-toluol erlangt. 6,7-Methylendioxyisochroman ist bekannt aus J. Org. Chem. Vol. 27 (1962), Seite 4338.

Es wurde nun gefunden, daß die Isochroman-derivate der Formel (I)

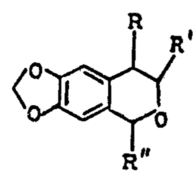

(I)

in welcher

R, R′ und R″ gleich oder verschieden sein können und für Wasserstoff oder Alkyl stehen, in Kombination mit

A) Carbamaten und/oder

B) Carbonsäureestern, einschließlich der natürlichen sowie synthetischen Pyrethroide, und/oder

C) Phosphorsäureestern und/oder

D) Halogenalkanen

eine besonders hohe insektizide und akarizide Wirkung ausweisen.

Die synergistische Wirkung der Verbindungen der allgemeinen Formel I zeigt sich bevorzugt bei

A) Carbamaten der allgemeinen Formel (II)

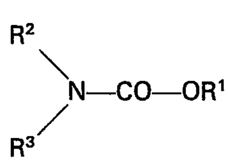

(II)

in welcher

$R^1$ für Aryl, einen Heterocyclus oder einen Oximrest steht,

$R^2$ für Wasserstoff oder einen Alkylrest mit 1 bis 4 Kohlenstoffatomen steht und

$R^3$ für Alkyl, Alkylcarbonyl mit 1 bis 6 Kohlenstoffatomen im Alkylrest, der gegebenenfalls auch durch Hydroxy oder Methylthio substituiert sein kann, oder den Rest —S—Z steht, wobei

Z für einen gegebenenfalls durch Halogen substituierten aliphatischen Rest mit 1 bis 4 Kohlenstoffatomen, insbesondere $CCl_3$ und $CF_3$ sowie für gegebenenfalls bevorzugt durch Nitril, Halogen, insbesondere Chlor, Methyl, Trihalogenmethyl, Trifluormethylmercapto oder Nitro substituierten Arylrest, insbesondere Phenyl, oder für Methoxycarbonylsteht.

Besonders bevorzugt sind Carbamate der Formel II, worin

$R^1$ für Phenyl oder Naphthyl steht, die gegebenenfalls substituiert sind durch Alkyl, Alkenyl, Alkoxy, Alkylmercapto oder Alkylthioalkyl mit jeweils 1 bis 6 Kohlenstoffatomen, Dialkylamino, Dialkenylamino mit bis zu 3 Kohlenstoffatomen je Alkyl- bzw. Alkenylteil, Halogen, insbesondere Chlor, Dioxolanyl oder den Rest —N=CH—N($C_{1-4}$-Alkyl)$_2$ steht.

Weiterhin sind besonders bevorzugt Carbamate der Formel II, worin

$R^1$ für 2,3-Dihydrobenzofuranyl, Benzodioxol, Benzothienyl, Pyrimidinyl oder Pyrazolyl steht, die gegebenenfalls durch Alkylgruppen mit 1 bis 4 Kohlenstoffatomen, insbesondere Methyl, oder durch Dialkylaminogruppen mit 1 bis 4 Kohlenstoffatomen je Alkylteil substituiert sind.

Weiterhin sind besonders bevorzugt Carbamate der allgemeinen Formel II, worin $R^1$ für einen Rest der Formel (II a)

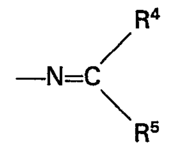

(II a)

steht, in welcher

$R^4$ und $R^5$ gleich oder verschieden sind und für Alkyl, Cycloalkyl, Alkenyl, Alkinyl, Alkoxy, Alkylmercapto, Alkoxycarbonyl, Carbonylamid, Alkylmercaptoalkyl, mit jeweils bis zu 5 Kohlenstoffatomen, Nitril, Aryl, insbesondere Phenyl, einen gegebenenfalls substituierten heterocyclischen Rest oder für Alkyl, das durch einen heterocyclischen Rest substituiert ist oder gemeinsam einen gegebenenfalls durch $C_{1-4}$-Alkyl substituierten Dioxolanyl- oder Dithiolanylrest stehen.

Besonders erwähnt seien folgende Carbamate der Formel II: 2-Methylphenyl-, 2-Äthylenphenyl-, 2-n-Propylphenyl-, 2-Methoxyphenyl-, 2-Äthoxyphenyl-, 2-iso-Propoxyphenyl-, 4-Methylphenyl-, 4-Äthylphenyl-, 4-n-Propylphenyl-, 4-Methoxyphenyl-, 4-Äthoxyphenyl-, 4-n-Propoxyphenyl-, 3,4,5-Trimethylphenyl-, 1-Naphthyl-, 2,3-Dihydro-2,2-dimethyl-7-benzofuranyl-, 2-(1,3-Dioxolan(2)yl-phenyl)-, bzw. 2,2-Dimethyl-1,3-benzodioxol(4)yl-N-methyl-carbamat und die entsprechenden -N-methyl-N-acetyl-, -N-methyl-N-trifluormethylthio, -N-methyl-N-dichlormonofluormethylthio- bzw. -N-methyl-N-dimethylaminothiocarbamate.

Weiter zeigt sich die synergistische Wirkung der Verbindungen der allgemeinen Formel I bevorzugt bei

B) Carbonsäureestern der allgemeinen Formel (II)

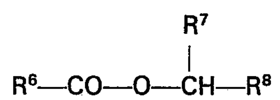

(III)

in welcher

$R^6$ für Alkyl, Aralkyl, Aryl oder Cycloalkyl steht, die gegebenenfalls substituiert sein können,

$R^7$ für Wasserstoff, Alkyl, Halogenalkyl, Alkenyl, Alkinyl, Nitril steht und

$R^8$ für Aryl oder einen Heterocyclus steht, oder gemeinsam mit $R^7$ einen gegebenenfalls substituierten Cyclopentenonring bildet.

Besonders bevorzugt sind Carbonsäureester, in denen $R^6$ für Alkyl mit 1 bis 6 Kohlenstoffatomen, das gegebenenfalls durch gegebenenfalls halogensubstituiertes Phenyl substituiert ist, Cyclopropyl, das gegebenenfalls durch Alkyl, Alkenyl, Halogenalkyl oder Halogenalkenyl mit jeweils bis zu 6 Kohlenstoffatomen substituiert ist, oder für Phenyl, das gegebenenfalls durch Halogen substituiert ist, steht. Bevorzugt sind Carbonsäureester, in denen $R^7$ für Wasserstoff, Alkyl mit 1 bis 6 Kohlenstoffatomen, Halogenalkyl mit 1 bis 4 Kohlenstoffatomen und bis zu 3 Halogenatomen, Nitril oder Äthinyl steht.

Weiter sind besonders bevorzugt Carbonsäureester, in denen $R^8$ für Phenyl steht, das gegebenenfalls durch $C_{1-4}$-Alkyl, Halogen, insbesondere Fluor oder Chlor, gegebenenfalls halogen- oder methylsubstituiertes Phenoxy, gegebenenfalls substituiertes Benzyl substituiert ist, ferner für Furanyl, Tetrahydrophthalimido, Benzodioxol, die gegebenenfalls durch Halogen, insbesondere Chlor, Alkyl oder Alkenyl mit bis zu 4 Kohlenstoffatomen oder Benzyl substituiert sind steht, und ferner für Cyclopentonon steht, das gegebenenfalls durch $C_{1-4}$-Alkyl, Furfuryl, $C_{2-5}$-Alkenyl substituiert ist.

Im einzelnen seien genannt:

Essigsäure - (1 - (3,4 - dichlorphenyl) - 2,2,2 - trichloräthyl)ester, 2,3,4,5 - Tetrahydrophthal - imidomethylchrysanthemat und (5 - Benzyl - 3 - furyl) - methyl - 2,2 - dimethyl - 3 - (2 - methyl - propenyl) - cyclopropancarboxylat. Weiter sind besonders bevorzugt die natürlich vorkommenden Pyrethroide.

Weiter zeigt sich die synergistische Wirkung der Verbindungen der allgemeinen Formel I bevorzugt bei

C) Phosphorsäureestern der allgemeinen Formel IV

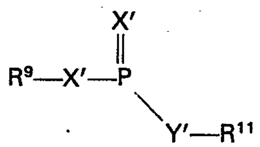

(IV)

in welcher

X' unabhängig voneinander für O oder S steht und

Y' für O, S, —NH— oder für eine direkte Bindung zwischen dem zentralen P-Atom und dem $R^{11}$ steht und

$R^9$ und $R^{10}$ gleich oder verschieden sind und für Alkyl oder Aryl stehen,

$R^{11}$ für Alkyl, Aryl, Heteroaryl, Aralkyl, Alkenyl, Dioxanyl, oder einen Oximrest oder für den gleichen Rest steht, an den es gebunden ist.

Besonders bevorzugt sind Phosphorsäureester, in denen

$R^9$ und $R^{10}$ gleich oder verschieden sind und für $C_{1-4}$-Alkyl oder Phenyl stehen,

$R^{11}$ für Alkyl mit 1 bis 4 Kohlenstoffatomen steht, das gegebenenfalls durch Halogen, Hydroxyl, Nitril, gegebenenfalls halogensubstituiertes Phenyl, Carbonylamid, Sulfonylalkyl, Sulfoxyalkyl, Carbonylalkyl, Alkoxy, Alkylmercapto, Alkoxycarbonyl, substituiert ist, für Alkenyl mit bis zu 4 Kohlenstoffatomen, das gegebenenfalls durch Halogen, gegebenenfalls halogen-substituiertes Phenyl oder Alkoxycarbonyl substituiert ist, oder für den Oximrest der allgemeinen Formel

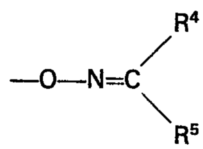

(II b)

wobei $R^4$ und $R^5$ die oben angegebene Bedeutung besitzen, insbesondere jedoch für Cyan oder Phenyl stehen,

$R^{11}$ steht ferner für Dioxanyl, das durch denselben Rest substituiert ist, an den $R^{11}$ gebunden ist, oder $R^{11}$ steht für den gleichen Rest, an den er gebunden ist, oder $R^{11}$ steht für Phenyl, das gegebenenfalls durch Methyl, Nitro, Nitril, Halogen, Methylthio substituiert ist; $R^{11}$ steht außerdem besonders bevorzugt für gegebenenfalls durch $C_{1-4}$-Alkyl, Halogen substituierte Heteroaromaten, wie Pyridin, Chinolin, Chinoxalin, Pyrimidin, Diazinon, Benzo-1,2,4-triazin.

im einzelnen seien genannt;

O,O-Dimethyl- bzw. O,O-Diäthyl-O-(2,2-dichlor- bzw. 2,2-dibromvinyl)-phosphorsäureester,
O,O-Diäthyl-O-(4-nitro-phenyl)-thionophosphorsäureester,
O,O-Dimethyl-O-(3-methyl-4-methylthio)-thionophosphorsäureester,
O,O-Dimethyl-O-(3-methyl-4-nitro)-thionophosphorsäureester,
O-Äthyl-S-n-propyl-O-(2,4-dichlorphenyl)-thionophosphorsäureester,
O-Äthyl-S-n-propyl-O-(4-methylthio-phenyl)-thionophosphorsäureester,
O,O-Dimethyl-S-(4-oxo-1,2,3-benzotriazin(3)yl-methyl)-thionothiolphosphorsäureester,
O-Methyl-O-(2-iso-propyl-6-methoxy-pyrimidin(4)yl-thionomethanphosphonsäureester,
O,O-Diäthyl-O-(2-iso-propyl-6-methyl-pyrimidin(4)yl)-thionophosphorsäureester,
O,O-Diäthyl-O-(3-chlor-4-methyl-cumarin(7)yl)-thionophosphorsäureester,
O,O-Dimethyl-2,2,2-trichlor-1-hydroxy-äthan-phosphonsäureester,
O,O-Dimethyl-S-(methylcarbamoylmethyl)-thionophosphorsäureester.

Weiter zeigt sich die synergistische Wirkung der Verbindungen der allgemeinen Formel I bevorzugt bei

D) Halogenalkanen der Formel

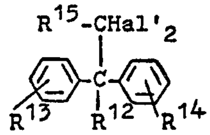

(V)

in welcher

Hal' für Chlor oder Brom und

$R^{12}$ für Wasserstoff oder Hydroxyl stehen,

$R^{13}$

und

$R^{14}$ gleich oder verschieden sind und für Halogen, Alkyl oder Alkoxy und

$R^{15}$ für Wasserstoff oder Halogen stehen.

Besonders bevorzugt sind Halogenalkane, in denen

$R^{12}$

und

$R^{13}$ Wasserstoff oder Hydroxyl bedeutet, und

$R^{14}$ für gleiches Halogen, Alkyl bzw. Alkoxy mit 1 bis 4 Kohlenstoffatomen je Alkyl- bzw. Alkoxyrest stehen und

$R^{15}$ Halogen bedeutet.

Im einzelnen seien genannt:
1,1,1-Trichlor-2,2-bis(4-chlor- bzw. -4-methoxphenyl)-äthan, 1,1,1-Trichlor-2-hydroxy-2,2-bis(4-chlorphenyl)-äthan und 1,1-Dichlor-2,2-bis(4-äthylphenyl)-äthan.

Überraschenderweise ist die insektizide und/oder akarizide Wirkung der erfindungsgemäßen Wirkstoffkombinationen wesentlich höher als die Wirkung der Einzelkomponenten bzw. die Summe der Wirkungen der Einzelkomponenten. Sie ist ferner wesentlich höher als die Wirkung der bereits bekannten Wirkstoffkombination aus 2-iso-Propoxy-phenyl-N-methyl-carbamat und Piperonylbutoxyd. Außerdem zeigen die erfindungsgemäß verwendbaren Benzodioxol-Derivate ausgezeichnete synergistische Wirksamkeit nicht nur bei einer Wirkstoffklasse, sondern bei Wirkstoffen aus den verschiedensten chemischen Stoffgruppen.

Somit stellen die erfindungsgemäßen Benzodioxol-Derivate enthaltenden synergistischen Mischungen eine wertvolle Bereicherung der Technik dar.

Die für die erfindungsgemäße Kombination zu verwendenden Synergisten sind durch die Formel I allgemein definiert. Vorzugsweise stehen darin jedoch

R, R' und R'' welche gleich oder verschieden sein können, für Wasserstoff oder geradkettiges wie auch verzweigtes Alkyl mit 1 bis 5, insbesondere 1 bis 3 Kohlenstoffatomen je Alkylrest.

Als Beispiele seien genannt:
6,7-Methylendioxy-isochroman, 1-Methyl-, 1-Äthyl-, 1-n-Propyl-, 1-iso-Propyl-, 3-Methyl-, 3-Äthyl-, 3-n-Propyl-, 3-iso-Propyl-, 4-Methyl-, 4-Äthyl-, 4-n-Propyl-, 4-iso-Propyl-, 1,3-Dimethyl-, 1,3-Diäthyl-, 1,3-Di-n-propyl-, 1,3-Di-iso-propyl-, 1,4-Dimethyl-, 1,4-Diäthyl-, 1,4-Di-n-propyl-, 1,4-Di-iso-propyl-, 3,4-Dimethyl-, 3,4-Diäthyl-, 3,4-Di-n-propyl-, 1-Methyl-3-äthyl-, 1-Methyl-3-n-propyl-, 1-Methyl-3-iso-propyl-, 1-Methyl-4-äthyl-, 1-Methyl-4-n-propyl-, 1-Methyl-4-iso-propyl-, 3-Methyl-4-äthyl-, 3-Methyl-4-n-propyl-, 3-Methyl-4-iso-propyl-, 1-Äthyl-3-methyl-, 1-Äthyl-3-n-propyl-, 1-Äthyl-3-iso-propyl-, 1-Äthyl-4-methyl-, 1-Äthyl-4-n-propyl-, 1-Äthyl-4-iso-propyl-, 3-Äthyl-4-methyl-, 3-Äthyl-4-n-propyl-, 3-Äthyl-4-iso-propyl-, 1-n-Propyl-3-methyl-, 1-n-Propyl-3-äthyl-, 1-n-Propyl-3-iso-propyl-, 1-n-Propyl-4-methyl-, 1-n-Propyl-4-äthyl-, 1-n-Propyl-4-iso-propyl-, 3-n-Propyl-4-methyl-, 3-n-Propyl-4-äthyl-, 3-n-Propyl-4-iso-propyl-, 1-iso-Propyl-3-methyl-, 1-iso-Propyl-3-äthyl-, 1-iso-Propyl-3-n-propyl-, 1-iso-Propyl-4-methyl-, 1-iso-Propyl-4-äthyl-, 1-iso-Propyl-4-n-propyl-, 3-iso-Propyl-4-methyl-, 3-iso-Propyl-4-äthyl-, 3-iso-Propyl-4-n-propyl-, 1,3,4-Trimethyl-, 1,3,4-Triäthyl-, 1,3,4-Tri-n-propyl-, 1-Äthyl-3,4-dimethyl-, 1-n-Propyl-3,4-dimethyl-, 1-iso-Propyl-3,4-dimethyl-, 1-Methyl-3,4-diäthyl-, 1-n-Propyl-3,4-diäthyl-, 1-iso-Propyl-3,4-di-n-propyl- 1-iso-Propyl-3,4-diäthyl-, 1-Methyl-3,4-di-n-propyl-, 1-Äthyl-3,4-di-n-propyl-, 1,3-Dimethyl-4-äthyl-, 1,3-Dimethyl-4-n-propyl-, 1,3-Dimethyl-4-iso-propyl-, 1,3-Diäthyl-4-methyl-, 1,3-Diäthyl-4-n-propyl-, 1,3-Diäthyl-4-iso-propyl-, 1,3-Di-n-propyl-4-methyl-, 1,3-Di-n-propyl-4-äthyl-, 1,3-Di-n-propyl-4-iso-propyl-, 1,3-Di-iso-propyl-4-methyl-, 1,3-Di-iso-propyl-4-äthyl-, 1,3-Di-iso-propyl-4-n-propyl-, 1,4-Dimethyl-3-äthyl-, 1,4-Dimethyl-3-n-propyl-, 1,4-Dimethyl-3-iso-propyl-, 1,4-Diäthyl-3-methyl-, 1,4-Diäthyl-3-n-propyl-, 1,4-Diäthyl-3-iso-propyl-, 1,4-Di-n-propyl-3-methyl-, 1,4-Di-n-propyl-3-äthyl-, 1,4-Di-n-propyl-3-iso-propyl-, 1,4-Di-iso-propyl-3-methyl-, 1,4-Di-iso-propyl-3-äthyl-, 1,4-Di-iso-propyl-3-n-propyl-, 3,4-Dimethyl-1-äthyl-, 3,4-Dimethyl-1-n-propyl-, 3,4-Dimethyl-1-iso-propyl-, 3,4-Diäthyl-1-methyl-, 3,4-Diäthyl-1-n-propyl-, 3,4-Diäthyl-1-iso-propyl-, 3,4-Di-n-propyl-1-methyl-, 3,4-Di-n-propyl-1-äthyl-, 1-Methyl-3-äthyl-4-n-propyl-, 1-Methyl-3-äthyl-4-iso-propyl-, 1-Methyl-3-n-propyl-4-äthyl-, 1-Methyl-3-n-propyl-4-iso-propyl-, 1-Methyl-3-iso-propyl-4-äthyl-, 1-Methyl-3-iso-propyl-4-n-propyl-, 1-Äthyl-3-methyl-4-n-propyl-, 1-Äthyl-3-methyl-4-iso-propyl-, 1-Äthyl-3-n-propyl-4-methyl-, 1-Äthyl-3-n-propyl-4-iso-propyl-, 1-Äthyl-3-iso-propyl-4-methyl-, 1-Äthyl-3-iso-propyl-4-n-propyl-, 1-n-Propyl-3-methyl-4-iso-propyl-, 1-n-Propyl-3-methyl-4-äthyl-, 1-n-Propyl-3-äthyl-4-iso-propyl-, 1-n-Propyl-3-äthyl-4-methyl-, 1-n-Propyl-3-iso-propyl-4-methyl-, 1-n-Propyl-3-iso-propyl-4-äthyl-, 1-iso-Propyl-3-methyl-4-äthyl-, 1-iso-Propyl-3-methyl-4-n-propyl-, 1-iso-Propyl-3-äthyl-4-methyl-, 1-iso-Propyl-3-äthyl-4-n-propyl-, 1-iso-Propyl-3-n-propyl-4-methyl- und 1-iso-Propyl-3-n-propyl-4-äthyl-6,7-methylendioxy-iso-chroman.

Die Isochroman-derivate werden erhalten, wenn mann Benzodioxolderivate der Formel (Ia)

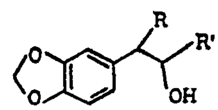

(Ia)

in welcher
R und R′ die oben angegebene Bedeutung haben,

mit Aldehyden Der Formel (Ib)

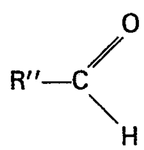

(Ib)

in welcher
R″ die oben angegebene Bedeutung hat,
gegebenenfalls in Gegenwart eines Katalysators und gegebenenfalls in Gegenwart eines Verdünnungsmittel, umsetzt.

Die als Ausgangsprodukte zu verwendenden Benzodioxolderivate sind durch Formel I a definiert. Vorzugsweise stehen darin
R und R′ welche gleich oder verschieden sein können, für Wasserstoff oder geradkettiges wie auch verzweigtes Alkyl mit 1 bis 5, insbesondere 1 bis 3 Kohlenstoffatomen je Alkylrest.

Als Beispiele seien genannt:
4-(2-Hydroxy-äthyl)-, 4-(2-Hydroxy-n-propyl)-, 4-(2-Hydroxy-n-butyl-, 4-(2-Hydroxy-n-pentyl)-, 4-(2-Hydroxy-iso-pentyl)-, 4-(1-Methyl-2-hydroxy-äthyl)-, 4-(1-Äthyl-2-hydroxy-äthyl)-, 4-(1-n-Propyl-2-hydroxy-äthyl)-, 4-(1-iso-Propyl-2-hydroxy-äthyl)-, 4-(1-Methyl-2-hydroxy-n-propyl)-, 4-(1-Äthyl-2-hydroxy-n-propyl)-, 4-(1-n-Propyl-2-hydroxy-n-propyl)-, 4-(1-iso-Propyl-2-hydroxy-n-propyl)-, 4-(1-Methyl-2-hydroxy-n-butyl)-, 4-(1-Äthyl-2-hydroxy-n-butyl)-, 4-(1-n-Propyl-2-hydroxy-n-butyl)-, 4-(1-iso-Propyl-2-hydroxy-n-butyl)-, 4-(1-Methyl-2-hydroxy-n-pentyl)-, 4-(1-Äthyl-2-hydroxy-n-pentyl)-, 4-(1-n-Propyl-2-hydroxy-n-pentyl)-, 4-(1-iso-Propyl-2-hydroxy-n-pentyl)-, 4-(1-Methyl-2-hydroxy-iso-pentyl)-, 4-(1-Äthyl-2-hydroxy-iso-pentyl)- und 4-(1-n-Propyl-2-hydroxy-iso-pentyl)-1,2-methylendioxybenzol.

Die Benzodioxolderivate der Formel I a sind teilweise bekannt (vergleiche DT—OS 2 624 693). Sie können hergestellt werden, indem man z.B. bekannte Verbindungen der Formel (I c)

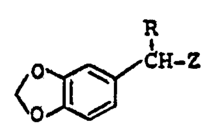

(I c)

worin
R die oben angegebene Bedeutung hat und
Z für COR′, COOR′ oder CN steht, wobei R′ die oben angegebene Bedeutung hat,
nach bekannten Methoden zu Verbindungen der Formel I a reduziert (vergleiche Ber. Deutsch. Chem. Ges. *41* (1908) S. 2751—2761).

In einem Spezialfall ($R = H$; $R' = CH_3$) geht man aus vom Isosafrol, oxydiert zum Epoxyd, lagert thermisch oder katalytisch um (Hoering, Ber. Dt. Chem. Ges. *38*, 2296—99; dto.

3464—3488 (1905)), reduziert das entstandene Piperonylmethylketon zum entsprechenden Alkohol.

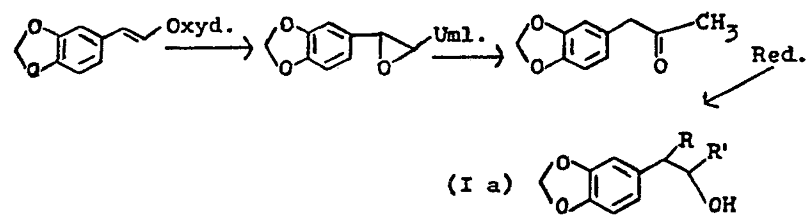


(I a)

R = H

R' = $CH_3$

Die weiter als Ausgangsstoffe zu verwendenden Aldehyde der Formel I b sind bekannt. Vorzugsweise steht in Formel I b

R'' für Wasserstoff oder geradkettiges wie auch verzweigtes Alkyl mit 1 bis 5, insbesondere 1 bis 3 Kohlenstoffatomen.

Als Beispiele seien genannt:
Formaldehyd, Acetaldehyd, Propionaldehyd, Butyraldehyd und iso-Butyraldehyd.

Das Verfahren zur Herstellung der erfindungsgemäß verwendbaren Verbindungen wird gegebenenfalls unter Verwendung geeigneter Verdünnungsmittel durchgeführt. Als solche kommen praktisch alle inerten organischen Solventien infrage. Hierzu gehören insbesondere aliphatische und aromatische, gegebenenfalls chlorierte, Kohlenwasserstoffe wie Benzol, Chlorbenzol, Dichlorbenzol, Toluol, Xylol, Methylenchlorid, Äthylenchlorid, Chloroform und Tetrachlorkohlenstoff.

Das Verfahren wird im allgemeinen unter Verwendung eines Katalysators durchgeführt. Als solcher wird vorzugsweise (gasförmiger) Chlorwasserstoff verwendet.

Die Reaktionstemperature kann innerhalb eines größeren Bereiches variiert werden. Im allgemeinen arbeitet man zwischen 0 und 80°C, vorzugsweise bei 10 bis 50°C. Die Umsetzung wird im allgemeinen bei Normaldruck durchgeführt.

Bei der Durchführung des Verfahrens setzt man im allgemeinen auf 1 Mol Hydroxyalkylbenzodioxolderivat der Formel I a zwischen 1,0 und 1,5 vorzugsweise zwischen 1,1 und 1,3 Mol Aldehyd der Formel I b ein. Im allgemeinen werden die Komponenten in einem der angegebenen Verdünnungsmittel vorgelegt und unter Durchleiten von Chlorwasserstoff mehrere Stunden bei den angegebenen Temperaturen gerührt. Die Aufarbeitung erfolgt durch Waschen mit Wasser, Trocknen der organischen Phase und Abziehen des Lösungsmittels im Vakuum. Die so erhaltenen Rohprodukte können durch Vakuumdestillation bzw. Umkristallisation gereinigt werden. Zur Charakterisierung dient der Schmelzpunkt.

Wie bereits erwähnt, zeigen Kombinationen der Isochromanderivate der Formel I mit Carbamaten, Carbonsäureestern, Phosphorsäureestern und Halogenalkanen eine hervorragende Wirkungssteigerung gegenüber den Einzelwirkstoffen bzw. gegenüber deren Summe.

Die Wirkstoffkombinationen eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hyginesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophaus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia keuhniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culux spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

In den erfindungsgemäßen Wirkstoffkombinationen liegt das Gewichtsverhältnis von Isochromanderivaten zu den anderen Wirkstoffen zwischen 0,1 : 10 und 10 : 0,1.

Die Wirkstoffkombinationen können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamide und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie

Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Beispiel A

$LT_{100}$ — Test

Testtiere: Musca domestica, Stamm Weymanns (gegen Carbamate und Phosphorsäureester resistent)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipetiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100%ige knock-down-Wirkung erforderlich ist. Wird die $LT_{100}$ nach 6 Stunden nicht erreicht, wird der %-Satz der knock down gegangenen Testtiere festgestellt.

Dabei zeigen z.B. die folgenden Verbindungen der Herstellungsbeispiele als Synergisten überlegene Wirkung gegenüber dem Stand der Technik: 1,2

## Herstellungsbeispiele

### Beispiel 1

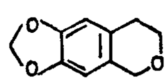

In eine Mischung aus 57 g (0,34 Mol) 4-(2-Hydroxy-äthyl). 1,2-methylendioxy-benzol, 12,8 g (0,425 Mol) Formaldehyd und 200 ml Äthylenchlorid wird unter Kühlen bei 20°C Chlorwasserstoff eingeleitet, bis eine klare Lösung entstanden ist. Die Lösung wird 6 Stunden bei 40°C unter Durchleiten von Chlorwasserstoff und über Nacht bei Raumtemperatur gerührt, mit Wasser und Natriumhydrogencarbonatlösung gewaschen und getrocknet. Nach Abziehen des Lösungsmittels im Vakuum wird das zurückbleibende Rohprodukt durch Vakuumdestillation gereinigt. Ausbeute 38 g (63% der Theorie), Siedepunkt 122°C/3 Torr. Das Produkt kristallisiert nach Anreiben mit Äther/Petroläther:Schmelzpunkt 87°C.

### Beispiel 2

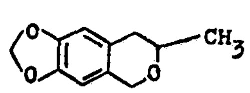

In eine Mischung aus 90 g (0,5 Mol) 4-(2-Hydroxy-n-propyl)-1,2-methylendioxy-benzol, 18,8 g (0,625 Mol) Formaldehyd und 250 ml Äthylenchlorid wird bei 15 bis 20°C Chlorwasserstoff eingeleitet, bis eine klare Lösung entstanden ist. Die Lösung wird 6 Stunden bei 40 bis 45°C gerührt, über Nacht stehen gelassen, in Eiswasser gegossen, mit Wasser und Natriumhydrogencarbonatlösung gewaschen

und getrocknet. Das Lösungsmittel wird im Vakuum destilliert und das Rohprodukt durch Vakuumdestillation gereinigt.

Ausbeute 41 g (43% der Theorie), Siedepunkt 136°C/3 Torr. Das Produkt kristallisiert nach Anreiben mit Äther/Petroläther: Schmelzpunkt 73°C.

**Patentansprüche**

1. Insektizide und akarizide Mittel, gekennzeichnet durch einen Gehalt an einer Wirkstoffkombination, bestehend aus Isochromanderivaten der Formel I

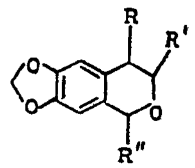

(I)

in welcher
R, R′ und R″ gleich oder verschieden sein können und für Wasserstoff oder Alkyl stehen

und
A) Carbamaten, und/oder
B) Carbonsäureestern, und/oder
C) Phosphorsäureestern und/oder
D) Halogenalkanen.

2. Insektizide und akarizide Mittel gemäß Anspruch 1, dadurch gekennzeichnet, daß in der Wirkstoffkombination das Gewichtsverhältnis von Isochromanderivaten zu den anderen Wirkstoffen zwischen 0,1 : 10 und 10 : 0,1 liegt.

3. Verfahren zur Bekämpfung von Insekten und Spinnentieren dadurch gekenzeichnet, daß man eine Wirkstoffkombination gemäß Anspruch 1 oder 2 auf Insekten und Spinnentieren und/oder ihren Lebensraum einwirken läßt.

4. Verwendung von Wirkstoffkombination gemäß Anspruch 1 oder 2 zur Bekämpfung von Insekten und Spinnentieren.

5. Verfahren zur Herstellung von insektiziden und akariziden Mitteln dadurch gekennzeichnet, daß man eine Wirkstoffkombination gemäß Anspruch 1 oder 2 mit Streckmitteln und/oder oberflächenaktiven Mitteln vermischt.

**Claims**

1. Insecticidal and acaricidal agents, characterised in that they contain an active compound combination consisting of isochromane derivatives of the formula I

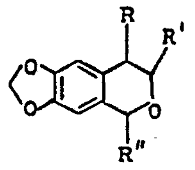

(I)

in which
R, R′ and R″ can be identical or different and represent hydrogen or alkyl
and
A) carbamates and/or
B) carboxylic acid esters and/or
C) phosphoric acid esters and/or
D) halogenoalkanes.

2. Inseticidal and acaricidal agents according to Claim 1, characterised in that in the active compound combination the weight ratio of isochromane derivatives to the other active compounds is between 0.1 : 10 and 10 : 0.1.

3. Process for combating insects and arachnidae, characterised in that an active compound combination according to Claim 1 or 2 is allowed to act on insects and arachnidae and/or their habitat.

4. Use of an active compound combination according to Claim 1 or 2 for combating insects and arachnidae.

5. Process for the preparation of insecticidal and acaricidal agents, characterised in that an active compound combination according to Claim 1 or 2 is mixed with extenders and/or surface-active agents.

## Revendications

1. Agents insecticides et acaricides, caractérisés en ce qu'ils contiennent une combinaison de substances actives consistant en dérivés d'isochromane de formule

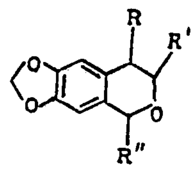

(I)

dans laquelle R, R' et R'' sont identiques ou différents et peuvent représenter l'hydrogène ou un groupe alkyle et

A) des carbamates et/ou
B) des esters carboxyliques et/ou
C) des esters phosphoriques et/ou
D) des halogénoalcanes.

2. Agents insecticides et acaricides selon la revendication 1, caractérisés en ce que le rapport pondéral des dérivés d'isochromane aux autres substances actives dans la combinaison de substances actives est compris enter 0,1 : 10 et 10 : 0,1.

3. Procédé pour la lutte contre les insectes et les araignées, caractérisé en ce que l'on fait agir sur les insectes et les araignées et/ou sur leur espace vital une combinaison de substances actives selon la revendication 1 ou 2.

4. Utilisation d'une combinaison de substances actives selon la revendication 1 ou 2 pour la lutte contre les insectes et les araignées.

5. Procédé pour la préparation d'agents insecticides et acaricides, caractérisé en ce qu'on mélange une combinaison de substances actives selon la revendication 1 ou 2 avec des agents diluants et/ou des agents tensioactifs.